# EUROPEAN PATENT SPECIFICATION

(11) **EP 2 116 531 B1**
(45) Date of publication and mention of the grant of the patent: **20.07.2011**
(21) Application number: 08720853.4
(22) Date of filing: 26.02.2008
(51) Int. Cl.: C07D 209/86, C09K 11/06, H01L 51/50

(54) **CBP COMPOUND**
CBP-VERBINDUNG
COMPOSÉ DE CBP

(30) Priority: 26.02.2007 JP 2007045663; 29.06.2007 JP 2007172497
(43) Date of publication of application: 11.11.2009
(73) Proprietor: Sumitomo Chemical Company, Limited, Tokyo 104-8260 (JP)
(72) Inventor: UETANI, Yasunori, Tsukuba-shi Ibaraki 305-0045 (JP); FUJIWARA, Jun, Ashiya-shi Hyogo 659-0055 (JP); NAKANISHI, Hirotoshi, Mishima-gun Osaka 618-0001 (JP)
(74) Representative: Duckworth, Timothy John
(86) International application number: PCT/JP2008/053265
(87) International publication number: WO 2008/105398

(56) References cited:
- WO-A1-2006/025290
- JP-A- 2005 071 909
- JP-A- 2005 533 873

## Description

### TECHNICAL FIELD

The present invention relates to a compound excellent in charge transporting properties.

### BACKGROUND ART

Recently, in the field of electronics, research and development have been actively conducted on organic functional devices which employ organic semiconductor compounds instead of inorganic materials such as silicon. Examples of the organic functional devices include organic electroluminescence devices (organic EL), organic transistors, organic solar cells, and the like. In particular, organic semiconductor compounds with high molecular weights are normally soluble in solvents, and thus can be formed into organic semiconductor layers by a coating method. Accordingly, the organic semiconductor compounds meet the requirement that production of devices be simplified. For this reason, polymer materials such as light emitting polymer materials, for example, have been proposed in recent years (Non Patent Document 1).

An organic semiconductor material used in such organic functional devices is required to have high charge transporting properties. High charge transporting properties bring advantages of, for example, lower driving voltage in an organic EL, higher operating speed in an organic transistor, and higher conversion efficiency in an organic solar cell.

### [Non Patent Document 1]

Advanced Materials Vol. 12 1737-1750 (2000)

### DISCLOSURE OF THE INVENTION

Conventional organic semiconductor materials, however, have a problem of insufficient charge transporting properties. For this reason, an object of the present invention is to provide a compound excellent in charge transporting properties and useful as an organic semiconductor material or the like.

First, the present invention provides a compound represented by the following Formula (1): 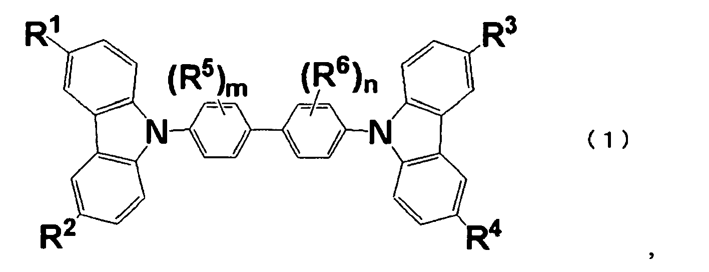 wherein R¹ to R⁴ each independently represent a group selected from the group consisting of a hydrogen atom, an alkyl group having 1 to 18 carbon atoms and a group represented by the following Formula (2): 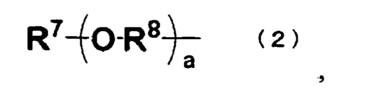 wherein R⁷ represents an alkyl group having 1 to 18 carbon atoms, R⁸ represents an alkylene group having 1 to 20 carbon atoms, a represents an integer of 1 to 5, and R⁸s are either the same as or different from each other when a is 2 or more); at least one of R¹ to R⁴ is a group represented by Formula (2); R⁵ and R⁶ each independently represent an alkyl group having 1 to 18 carbon atoms; and m and n each independently represent an integer of 0 to 4).

Second, the present invention provides a charge transporting material, the material including the compound.

Third, the present invention provides a composition including: at least one polymer material selected from a charge transporting material and a light emitting material; and the compound.

Fourth, the present invention provides an organic transistor comprising a charge transporting layer or an active layer made of the composition.

Fifth, the present invention provides an organic electroluminescence device comprising a charge transporting layer or a light emitting layer made of the composition.

Sixth, the present invention provides an organic solar cell comprising a charge transporting layer or an active layer made of the composition.

The compound of the present invention is excellent in charge transporting properties, when used as a material for an electroluminescence light emitting device, an organic transistor, a solar cell, or the like. Furthermore, the compound of the present invention is normally excellent in coating properties (i.e. , the compound hardly aggregates when dissolved or dispersed in a solvent) and excellent in driving voltage.

### BEST MODES FOR CARRYING OUT THE INVENTION

### <Compound>

A compound of the present invention is a compound represented by the following Formula (1): 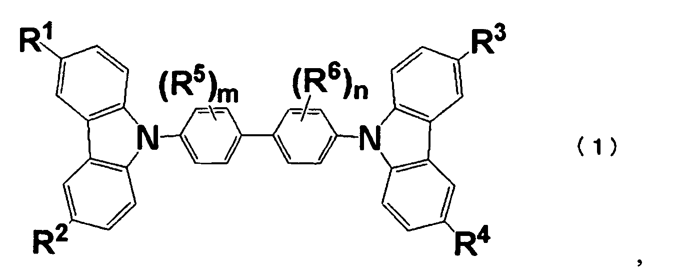 wherein where R¹ to R⁴ each independently represent a group selected from the group consisting of a hydrogen atom, an alkyl group having 1 to 18 carbon atoms and a group represented by the following Formula (2): 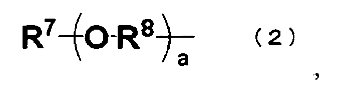 wherein R⁷ represents an alkyl group having 1 to 18 carbon atoms, R⁸ represents an alkylene group having 1 to 20 carbon atoms, a represents an integer of 1 to 5, and R⁸s are either the same as or different from each other when a is 2 or more); at least one of R¹ to R⁴ is a group represented by Formula (2); R⁵ and R⁶ each independently represent an alkyl group having 1 to 18 carbon atoms; and m and n each independently represent an integer of 0 to 4) .

All of R¹ to R⁴ in the compound of Formula (1) may be a group represented by Formula (2). Preferably, one or two of R¹ to R⁴, for example, R¹ and R⁴ or R² and R³ in the compound of Formula (1) are a group represented by Formula (2). More preferably, one of R¹ to R⁴ in the compound of Formula (1) is a group represented by Formula (2).

Examples of the alkyl group having 1 to 18 carbon atoms represented by R¹ to R⁴ and R⁷ include linear or branched ones such as a methyl group, an ethyl group, an n-propyl group, an isopropyl group, an n-butyl group, an isobutyl group, a sec-butyl group, a tert-butyl group, an n-pentyl group, an n-hexyl group, an n-octyl group, an isooctyl group, an n-decyl group, an n-dodecyl group, an n-pentadecyl group, and an n-octadecyl group. The alkyl group is preferably an alkyl group having 1 to 10 carbon atoms, more preferably an alkyl group having 2 to 8 carbon atoms, and even more preferably an alkyl group having 3 to 6 carbon atoms.

Examples of the alkylene group having 1 to 20 carbon atoms represented by R⁸ include linear or branched ones such as a methylene group, an ethylene group, a propylene group, an i-propylene group, a butylene group, an i-butylene group, a pentylene group, a hexylene group, a heptylene group, an octylene group, a 2-ethylhexylene group, a nonylene group, a decylene group, a 3,7-dimethyloctylene group, and a laurylene group. The alkylene group is preferably an alkylene group having 1 to 10 carbon atoms, more preferably an alkylene group having 2 to 8 carbon atoms, and even more preferably an alkylene group having 3 to 6 carbon atoms.

Preferably, a represents an integer of 2 to 4, and more preferably 3.

Examples of the alkyl group having 1 to 18 carbon atoms represented by R⁵ and R⁶ include linear or branched ones such as a methyl group, an ethyl group, an n-propyl group, an isopropyl group, an n-butyl group, an isobutyl group, a sec-butyl group, a tert-butyl group, an n-pentyl group, an n-hexyl group, an n-octyl group, an isooctyl group, an n-decyl group, an n-dodecyl group, an n-pentadecyl group, and an n-octadecyl group. The alkyl group is preferably an alkyl group having 1 to 8 carbon atoms.

Preferably, m and n each represent an integer of 0 or 1.

The specific examples of the compound represented by Formula (1) are shown below.

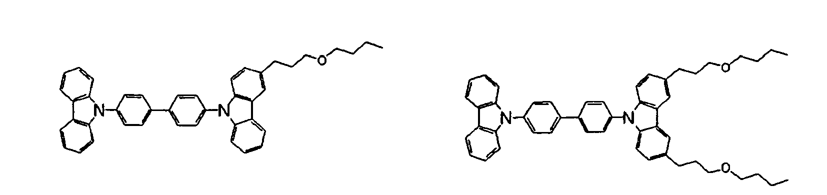

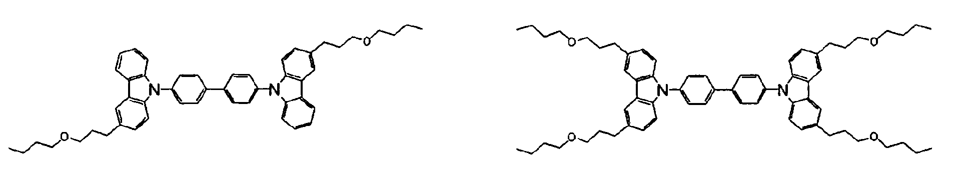

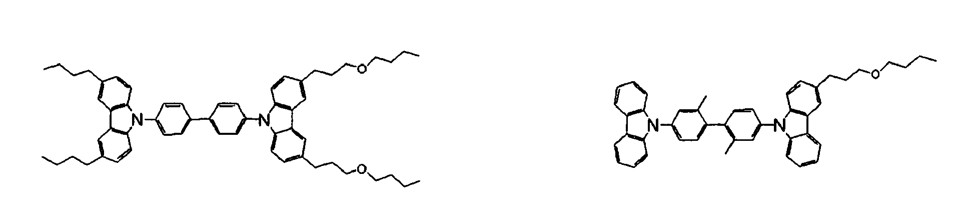

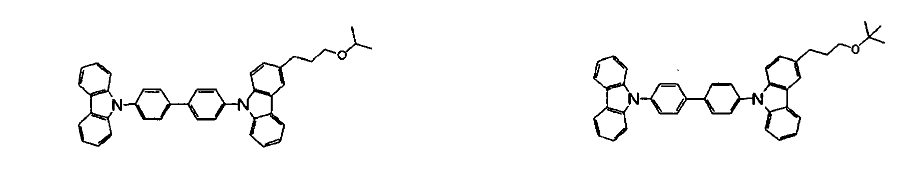

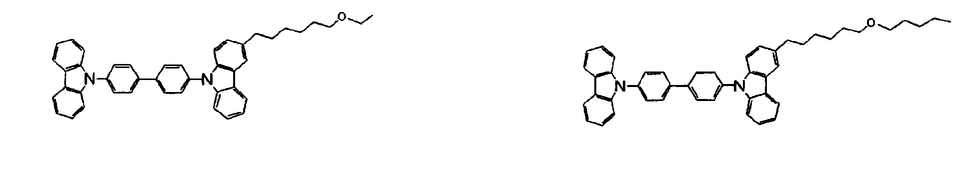

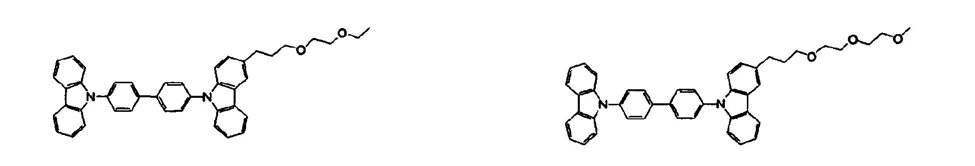

The compounds of the present invention can be used as a charge transporting material, alone or in combination with other components.

### -Manufacturing Method-

Next, manufacturing methods of the compound represented by Formula (1) will be described. Note that the compound of the present invention may be manufactured by a method other than the methods below.

The compound represented by Formula (1) can be obtained by, for example, condensation of a hydroxyalkylated product of 4,4'-bis(9-carbazoyl)-biphenyl (hereinafter, referred to as "CBP") with an alkyl halide. Specifically, the compound can be synthesized by a method known as the Williamson method by which a desired ether bond is formed as follows. The hydroxyl group of the hydroxyalkylated product is converted into a highly reactive alkoxide by an alkali such as a metal hydride; and the highly reactive alkoxide is caused to nucleophilically attack the alkyl halide for substitution. The alkali used in this reaction is not limited to metal hydrides such as sodium hydride and potassium hydride, but instead includes metal carbonates such as potassium carbonate and sodium carbonate. After the reaction, work-up operations are performed so that the reaction mixture is poured into water, extracted with an organic solvent, and the obtained organic phase is concentrated. Thus, the compound can be obtained. The compound may be further purified by recrystallization, chromatography, or the like. Note that the synthesis of the hydroxyalkylated product of CBP, for example, 3-(3-hydroxypropyl)CBP and 3-(6-hydroxyhexyl)CBP, can be achieved as follows. Specifically, in the presence of a Lewis acid such as AlCl₃ or FeBr₃, addition reaction takes place on CBP with an alkyl bromide, an alkyl iodide, or the like, having its terminal hydroxyl group protected by a trialkylsilyl group, an ester group, or the like, in an organic solvent such as nitrobenzene or triethyl phosphate. Thus, CBP is alkylated by a method known as the Friedel-Crafts reaction. Purification and isolation by, for example, column chromatography are performed if necessary, and further the protecting group of the hydroxyl group is removed.

In these condensation reactions, the above-described hydroxyalkylated product is ordinarily used in a dissolved form in a solvent. Ordinarily, this solvent is preferably in a liquid state at -40 to 40°C under a pressure of 1.0×10⁵ Pa.

Examples of the solvent include chloroform, methylene chloride, dichloroethane, tetrahydrofuran, toluene, xylene, mesitylene, tetralin, decalin, n-butylbenzene, chlorobenzene, o-dichlorobenzene, and the like. When such a solvent is used, 0.1% by weight or more of the hydroxyalkylated product can be dissolved in the solvent, although the amount depends on the kind of the compound, the composition, and the like. Note that these solvents may be used alone or in combination with two or more kinds thereof.

When the hydroxyalkylated product is dissolved in the solvent, the amount of the solvent is generally approximately 1000 to 100000 parts by weight per 100 parts by weight of the compound.

### <Composition>

The composition according to the present invention includes: at least one polymer material selected from a charge transporting material and a light emitting material; and the above-described compound. This composition can be used also as a light emitting polymer. In other words, the light emitting polymer is made of the composition of the present invention.

The charge transporting material refers to a material having a charge transporting ability. The light emitting material refers to a material having a light emitting ability. Furthermore, the polymer material may be a material having both the charge transporting ability and the light emitting ability. An example of these polymer materials is a conjugated polymer. The conjugated polymer refers to a polymer compound in which delocalized n electron pairs exist along the main chain of the polymer compound. Unpaired electrons or lone pair electrons instead of double bonds may take part in the resonance, as delocalized electrons in the delocalized n electron pairs. Specific examples of the conjugated polymer include: polyarylenes such as polyfluorene [for example, Japanese Journal of Applied Physics (Jpn. J. Appl. Phys.) Vol. 30, page L1941 (1991)], poly(para-phenylene) [for example, Advanced Materials (Adv. Mater.) Vol. 4, page 36 (1992)], polypyrrole, polypyridine, polyaniline and polythiophene; poly(arylene-vinylene)s (for example, WO98/27136) such as poly(para-phenylene-vinylene) and poly(thienylene-vinylene); poly(phenylene sulfide); polycarbazole; and the like. Examples of reviews include the aforementioned "Advanced Materials Vol. 12 1737-1750 (2000)", "Organic EL Display Technology, Monthly DISPLAY, December 2001 edition, Special issue, pages 68 to 73", and the like. These conjugated polymers may have substituents.

In light of film formability and solubility in a solvent, the polystyrene-equivalent number average molecular weight of the conjugated polymer is preferably approximately 10³ to 10⁸, and more preferably approximately 10³ to 10⁶. Meanwhile, the polystyrene-equivalent weight average molecular weight of the conjugated polymer is preferably 10³ to 1×10⁸, and more preferably 1×10³ to 1×10⁶_{.}

The conjugated polymer can be synthesized as follows. Specifically, monomers are synthesized each of which has a functional group suitable for the polymerization reaction to be used; thereafter, the monomer is dissolved in an organic solvent, if necessary; and the monomers are polymerized by such a polymerization method as a publicly-known aryl coupling method using an alkali, an appropriate catalyst and an appropriate ligand.

The polymerization method involving the aryl coupling is not particularly limited, and examples thereof include: a polymerization method by Suzuki coupling reaction in which a monomer having a functional group suitable for the above-described polymerization reaction, such as a boric acid residue (i.e., -B(OH)₂) or a borate ester residue (e.g.: 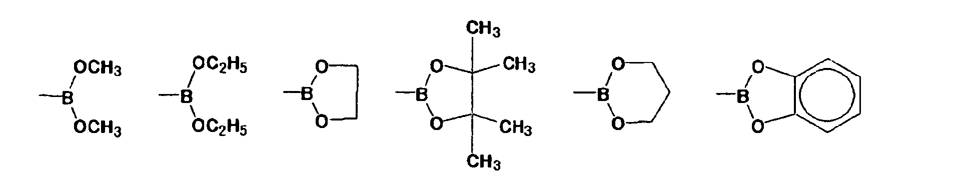 and the like) and a monomer having, as a functional group, a halogen atom such as a bromine atom, an iodine atom or a chlorine atom or a sulfonate group such as a trifluoromethanesulfonate group or a p-toluenesulfonate group are polymerized in the presence of an inorganic base such as sodium carbonate, potassium carbonate, cesium carbonate, tripotassium phosphate, or potassium fluoride or an organic base such as tetrabutylammonium fluoride, tetrabutylammonium chloride, tetrabutylammonium bromide, or tetraethylammonium hydroxide, by using a catalyst including a palladium or nickel complex such as [tetrakis(triphenylphosphine)]palladium, [tris(dibenzylideneacetone)]dipalladium, palladium acetate, bis(triphenylphosphine)palladium dichloride or bis(cyclooctadiene)nickel, and, if necessary, an additional ligand such as triphenylphosphine, tri(2-methylphenyl)phosphine, tri(2-methoxyphenyl)phosphine, diphenylphosphinopropane, tri(cyclohexyl)phosphine or tri(tert-butyl)phosphine; a polymerization method by Yamamoto coupling reaction in which monomers each having a halogen atom or a sulfonate group such as a trifluoromethanesulfonate group are reacted with each other by using a catalyst including a zero-valent nickel complex, such as bis (cyclooctadiene) nickel, and a ligand such as bipyridyl, or by using a catalyst including a Ni complex such as [bis(diphenylphosphino)ethane]nickel dichloride or [bis(diphenylphosphino)propane]nickel dichloride, and, if necessary, an additional ligand such as triphenylphosphine, diphenylphosphinopropane, tri(cyclohexyl)phosphine or tri (tert-butyl) phosphine, as well as a reducing agent such as zinc or magnesium, under a dehydrated condition, if necessary; a polymerization method by Kumada-Tamao coupling reaction in which a compound having a magnesium halide group and a compound having a halogen atom are reacted for polymerization through aryl coupling reaction by using a Ni catalyst such as [bis(diphenylphosphino)ethane]nickel dichloride and [bis(diphenylphosphino)propane]nickel dichloride, under a dehydrated condition; a method in which polymerization is performed by using an oxidizing agent such as FeCl₃ while hydrogen atoms are utilized as functional groups; a method in which oxidative polymerization is performed electrochemically; and the like.

The solvent used for the reaction should be selected depending on a polymerization reaction to be used, solubilities of the monomers and the polymer, and the like. Specific examples of the solvent include: organic solvents such as tetrahydrofuran, toluene, 1,4-dioxane, dimethoxyethane, N,N-dimethylacetamide, N, N-dimethylformamide, and a mixture solvent of two or more kinds thereof; or a two phase solvent system of any of these organic solvents with water.

For the Suzuki coupling reaction, preferably used are organic solvents such as tetrahydrofuran, toluene, 1,4-dioxane, dimethoxyethane, N,N-dimethylacetamide, N,N-dimethylformamide, and a mixture solvent of two or more kinds thereof; or a two phase solvent system of any of these organic solvents with water. Generally, the reaction solvent is preferably deoxygenized to suppress the side reactions.

For the Yamamoto coupling reaction, preferably used are organic solvents such as tetrahydrofuran, toluene, 1,4-dioxane, dimethoxyethane, N,N-dimethylacetamide, N,N-dimethylformamide, and a mixture solvent of two or more kinds thereof. Generally, the reaction solvent is preferably deoxygenized to suppress the side reactions.

Of these aryl coupling reactions, the Suzuki coupling reaction and the Yamamoto coupling reaction are preferable in light of reactivity, and the Suzuki coupling reaction and the Yamamoto coupling reaction which employs a zero-valent nickel complex are more preferable. More specifically, regarding the polymerization by the Suzuki coupling, publicly-known methods described, for example, in Journal of Polymer Science: Part A: Polymer Chemistry, Vol. 39, 1533-1556 (2001) can be used as a reference. Regarding the polymerization by the Yamamoto coupling, publicly-known methods described, for example, in Macromolecules 1992, 25, 1214-1223 can be used as a reference.

The reaction temperature for these reactions is not particularly limited, as long as the temperature is in a temperature range within which the reaction solution keeps its liquid state. However, the lower limit of the temperature is preferably -100°C, more preferably -20°C, and particularly preferably 0°C in light of reactivity, while the upper limit of the temperature is preferably 200°C, more preferably 150°C, and particularly preferably 120°C, in light of the stability of the above-described conjugated polymer and the stability of the compound represented by Formula (1).

Isolation of the conjugated polymer can be performed in accordance with publicly-known methods. For example, the reaction solution is poured into a lower alcohol such as methanol, and the deposited precipitate is filtered and dried. Thus, the conjugated polymer can be obtained. When the purity of the obtained conjugated polymer is low, the obtained conjugated polymer can be purified by an ordinary method such as recrystallization, continuous extraction using a Soxhlet extractor, or column chromatography.

The composition according to the present invention may contain a solvent in addition to the above-described compound and the polymer material. In such a case, an organic semiconductor layer can be formed by a coating method. Note that, when the composition contains a solvent, the composition is preferably in a solution state. The composition according to the present invention may include other components, as long as the components do not impair the effect of the present invention.

Examples of the solvent include alcohols (methanol, ethanol, isopropyl alcohol, and the like), ketones (acetone, methyl ethyl ketone, and the like), organochlorides (chloroform, 1,2-dichloroethane, and the like), aromatic hydrocarbons (benzene, toluene, xylene, and the like), aliphatic hydrocarbons (normal hexane, cyclohexane, and the like), amides (dimethylformamide and the like), sulfoxides (dimethyl sulfoxide and the like), and so forth. These solvents may be used alone or in combination with two or more kinds.

The content of the compound in the composition is not particularly limited; however, the content is preferably 0.1 to 10000 parts by weight per 100 parts by weight of the polymer material.

Note that the composition according to the present invention may include the above-described compound alone or in combination with two or more kinds and the above-described polymer material alone or in combination with two or more kinds.

For example, the compound and the composition according to the present invention are useful for an organic electroluminescence device (for example, as a material for a charge transporting layer, a light emitting layer, or the like), an organic transistor and a solar cell (for example, as a material for an active layer or the like), and other applications.

### <Organic Electroluminescence Device>

An organic electroluminescence device according to the present invention comprises a charge transporting layer or a light emitting layer made of the above composition. Specifically, the organic electroluminescence device comprises, for example: electrodes of an anode and a cathode; and a layer made of the composition and provided between the electrodes to serve as a charge transporting layer or a light emitting layer. Examples of the organic electroluminescence device include ones with the following layer constructions:
a) Anode/hole injection layer (hole transporting layer)/light emitting layer/cathode;
b) Anode/light emitting layer/electron injection layer (electron transporting layer)/cathode; and
c) Anode/hole injection layer (hole transporting layer)/light emitting layer/electron injection layer (electron transporting layer)/cathode.

The light emitting layer made of the composition according to the present invention is preferably formed by a coating method. The coating method is preferable because the manufacturing process can be simplified and because the productivity is excellent. Examples of the coating method include a casting method, a spin coating method, a bar coating method, a blade coating method, a roll coating method, a nozzle coating method, a capillary coating method, gravure printing, screen printing, an ink-jet method, and the like. In all of these coating methods, the composition (in a solution state) is prepared as a coating liquid. The coating liquid is applied on a desired layer or electrode, and then dried. Thus, a desired layer or film can be formed.

### <Organic Solar Cell>

An organic solar cell according to the present invention comprises a layer made of the above composition. Specifically, the organic solar cell comprises, for example: electrodes of an anode and a cathode one of which is transparent or translucent; and a layer made of the composition and provided between the electrodes to serve as a charge transporting layer or an active layer.

### <Organic Transistor>

An organic transistor according to the present invention comprises: a source electrode; a drain electrode; a gate electrode placed on an insulating layer, which is in contact with an active layer and interposed between the gate electrode and the electrodes; and a layer made of the above composition and being in contact with the source electrode and the drain electrode to serve as a charge transporting layer or the active layer.

### Examples

Hereinafter, Examples will be illustrated to describe the present invention in further detail; however, the present invention is not limited thereto.

### -Molecular Weight Determination Method-

In Examples, number average molecular weight (Mn) and weight average molecular weight (Mw) were determined by gel permeation chromatography (GPC) as a polystyrene equivalent value. Specifically, the determination was performed at 40°C with a GPC system (manufactured by TOSOH CORPORATION, trade name: HLC-8220GPC) using three serially connected columns of TSKgel Super HM-H (manufactured by TOSOH CORPORATION), while tetrahydrofuran serving as the eluent was flowing at a flowing rate of 0.5 ml/min. A differential refractive index detector was used as the detector.

### <Synthesis Example 1> (Synthesis of Polymer Compound 1)

1.72 g of triscaprylylmethylammonium chloride (manufactured by Sigma-Aldrich Co., trade name: Aliquat 336), 6.2171 g of Compound A represented by the following formula: 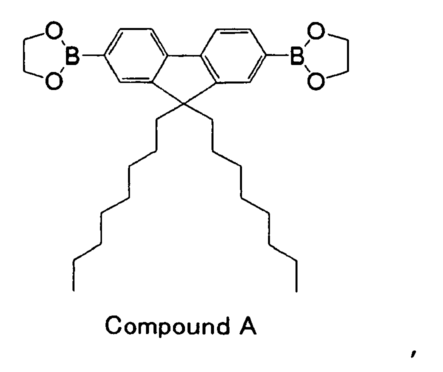 0.5085 g of Compound B represented by the following formula: 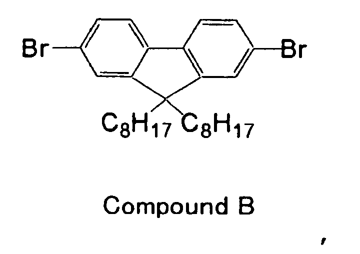 6.2225 g of Compound C represented by the following formula: 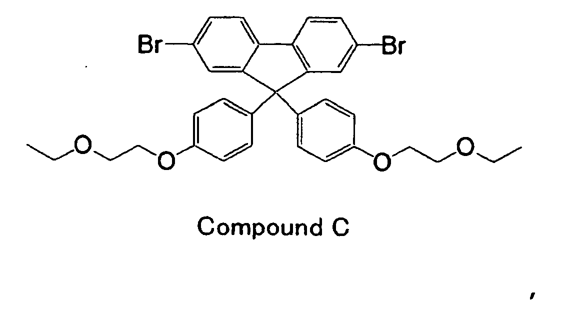 and 0.5487 g of Compound D represented by the following formula: 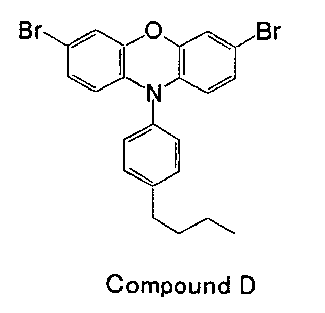 were placed in a 500-ml four-necked flask and the air inside the flask was replaced by nitrogen. Then, 100 ml of toluene was added, and 7.6 mg of dichlorobis(triphenylphosphine)palladium(II) and 24 ml of a sodium carbonate aqueous solution were added thereto. After stirring for 3 hours under reflux, 0.40 g of phenyl boric acid was added, and the mixture was stirred overnight. A sodium N,N-diethyldithiocarbamate aqueous solution was added, and then the mixture was stirred under reflux for 3 hours. Phases of the obtained reaction liquid were separated from each other. The organic phase was washed with an acetic acid aqueous solution and with water. Thereafter, the organic phase was added dropwise to methanol. As a result, a precipitate was formed. The resultant precipitate was filtered and dried under vacuum. The precipitate was dissolved in toluene, and the solution was passed through a silica gel-alumina column, which was then washed with toluene. The obtained toluene solution was added dropwise to methanol, and a precipitate was formed. The resultant precipitate was filtered and dried under vacuum. The precipitate was dissolved in toluene, and the solution were added dropwise to methanol, and a precipitate was formed. The resultant precipitate was filtered and dried under vacuum. Thus, 7.72 g of Polymer Compound 1 (conjugated polymer) was obtained. The polystyrene-equivalent number average molecular weight Mn of Polymer Compound 1 was 1.2x105, and the polystyrene-equivalent weight average molecular weight Mw thereof was 2.9×10⁵.

### <Synthesis Example 2> (Synthesis of Polymer Compound 2)

40.18 g of triscaprylylmethylammonium chloride (manufactured by Sigma-Aldrich Co., trade name: Aliquat 336), 234.06 g of Compound A represented by the following formula: 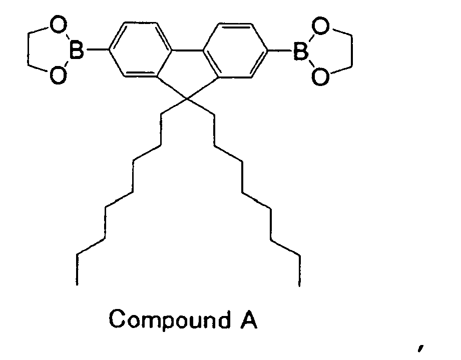 172.06 g of Compound E represented by the following formula: 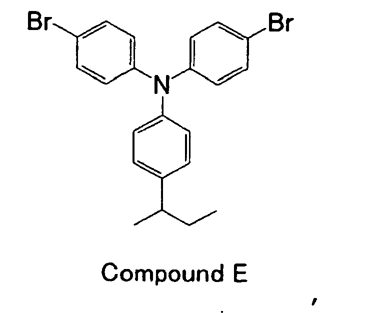 and 28.5528 g of Compound F represented by the following formula: 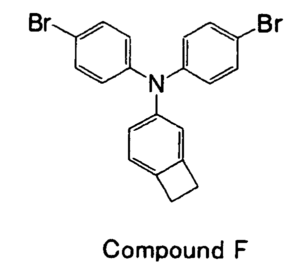 were placed in a 5-L separable flask and the air inside the flask was replaced by nitrogen. Then, 2620 g of argon bubbled toluene was added thereto. With stirring, additional 30-minute bubbling was performed. Thereafter, 99. 1 mg of palladium acetate and 937.0 mg of tris(o-tolyl)phosphine were added, which were then washed with 158 g of toluene. The mixture was heated to 95°C. After dropwise addition of 855 g of a 17.5% by weight sodium carbonate aqueous solution, the bath temperature was raised to 110°C, and the mixture was stirred for 9.5 hours. Thereafter, 5.39 g of phenyl boric acid dissolved in 96 ml of toluene were added thereto, and the mixture was stirred for 14 hours. After addition of 200 ml of toluene, the layers of the reaction liquid were separated from each other. The organic layer was washed twice with 850 ml of 3% by weight acetic acid aqueous solution. Further, 850 ml of water and 19.89 g of sodium N,N-diethyldithiocarbamate were added thereto, and the mixture was stirred for 4 hours.

After phase separation, the liquid was passed through a silica gel-alumina column, which was then washed with toluene. The obtained toluene solution was added dropwise to 50 L of methanol, and a precipitate was formed. The resultant precipitate was washed with methanol, and dried under vacuum. Thereafter, the precipitate was dissolved in 11 L of toluene. The obtained toluene solution was added dropwise to 50 L of methanol, and a precipitate was formed. The resultant precipitate was filtered and dried under vacuum. Thus, 278.39 g of Polymer Compound 2 were obtained. The polystyrene-equivalent number average molecular weight Mn of Polymer Compound 2 was 7.7×10⁴, and the polystyrene-equivalent weight average molecular weight Mw thereof was 3.8×10⁵.

### <Example 1> (Synthesis of Charge Transporting Compound H)

1.63 g (3.0 mmol) of Compound G (purchased from Tokyo Chemical Industry Co., Ltd.) represented by the following formula: 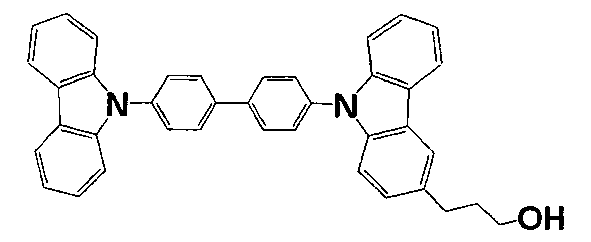 and 16.3 g of N,N-dimethylformamide were stirred in a flask, Then, 0.24 g (6.0 mmol) of sodium hydride (60% oil dispersion) was added, and the mixture was stirred for 30 minutes. Then 0.82 g (6.0 mmol) of n-butyl bromide was added thereto, and the mixture was stirred for 18 hours. Thereafter, the obtained reaction liquid was poured into 100 ml of water. Extraction was performed twice with 100 ml of chloroform, and the oil layer was washed twice with 100 ml of water. After the oil layer was concentrated by using an evaporator, the residue was purified by silica gel chromatography (eluent was chloroform:hexane = 1:1 (volume ratio)). Thus, 1.70 g (Yield: 94.4%) of Charge Transporting Compound H represented by the following formula: 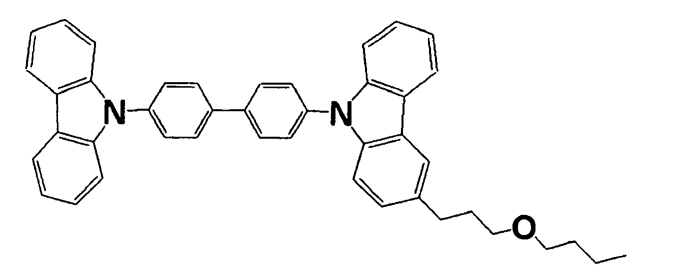 was obtained.
¹H-NMR (270 MHz, CDCl₃):
δ 0.93 (t, 3H), 1.43 (m, 2H), 1.59 (m, 2H), 2.02 (m, 2H), 2.88 (m, 2H), 3.45 (m, 4H), 7.25-7.34 (m, 5H), 7.38-7.51 (m, 6H), 7.70 (d, 4H), 7.90 (d, 4H), 7.97 (s, 1H), 8.11-8.20 (m, 3H)

### <Example 2> (Synthesis of Charge Transporting Compound J)

Compound I (purchased from Tokyo Chemical Industry Co., Ltd.) (1.17 g, 2.0 mmol) represented by the following formula: 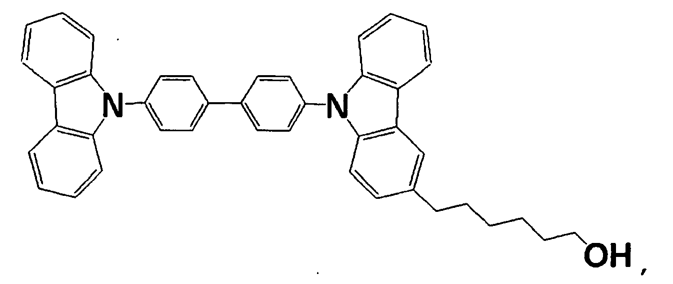 11.7 g of N,N-dimethylformamide, and 0.16 g (4.0 mmol) of sodium hydride (60% oil dispersion) were stirred in a flask for 30 minutes. The temperature was raised to 50°C. Then, 0.82 g (6.0 mmol) of n-butyl bromide was added dropwise thereto, and the mixture was stirred for 7 hours. After the mixture was cooled to room temperature, 0.16 g of sodium hydride (60% oil dispersion) was added thereto. Then, 0.82 g (6.0 mmol) of n-butyl bromide was added dropwise thereto, and the mixture was stirred for 3.5 hours. Further, 0.16 g of sodium hydride (60% oil dispersion) was added thereto, and the mixture was stirred at room temperature overnight. The reaction liquid was poured into 100 ml of water, and extraction was performed twice with 100 ml of chloroform. The obtained organic layer was washed twice with 100 ml of water. After the organic layer was concentrated by using an evaporator, the obtained residue was purified by silica gel chromatography (hexane/chloroform = 2:1 (volume ratio)). Thus, 1.1 g (Yield: 86.7%) of Compound J represented by the following formula: 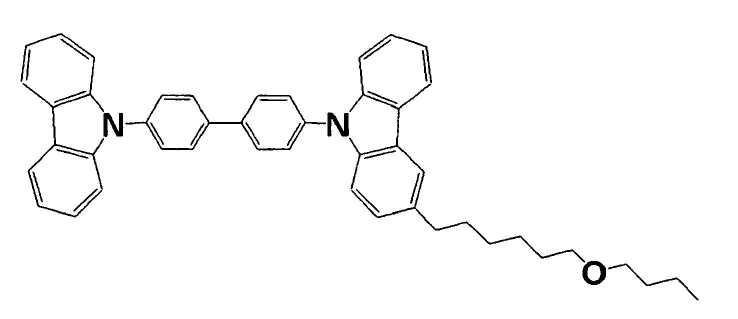 was obtained.
¹H-NMR (270 MHz, CDCl₃):
δ 1.91 (t, 3H), 1.20-1.80 (m, 12H), 2.82 (t, 2H), 3,40 (t, 4H), 7.20-7.35 (m, 4H), 7.35-7.53 (m, 7H), 7.70 (m, 4H), 7.85-8.00 (m, 5H), 8.10-8.20 (m, 3H)

### <Example 3> (Synthesis of Charge Transporting Compound L)

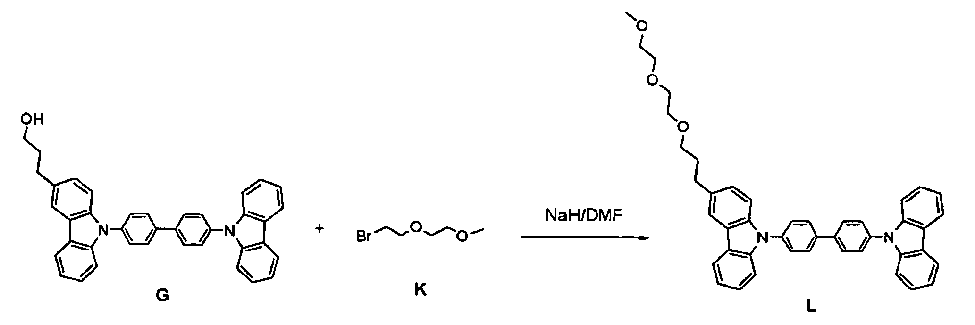

Under nitrogen atmosphere, Compound G (0.17 g, 0.31 mmol) and 9.5 g of N,N-dimethylformamide were placed in a 50-ml three-necked flask, and 0.03 g (0.63 mmol) of sodium hydride (60% oil dispersion) was added thereto. Then, the mixture was stirred for 30 minutes. The temperature was raised to 50°C. Then, 0.07 g (0.41 mmol) of the above-described halogen compound K was added dropwise thereto, and the mixture was stirred for 7 hours. The reaction liquid was poured into 20 ml of water, and extraction was performed twice with 20 ml of ethyl acetate. The obtained organic layer was washed twice with 20 ml of water. After the organic layer was concentrated by using an evaporator, the obtained residue was purified by silica gel chromatography (10 g of silica gel was used; elution was performed by using 200 ml of hexane/ethyl acetate =2:1 (volume ratio) ; and further elution was performed by using 200 ml of ethyl acetate). Thus, 0.14 g (Yield: 69.3%) of Compound L was obtained. ¹H-NMR (270 MHz, CDCl₃):
δ 2.01 (m, 2H), 2.89 (t, 2H), 3,37 (s, 3H), 3.48-3.58 (m, 4H), 3.58-3.70 (m, 6H), 7.20-7.35 (m, 4H), 7.35-7.53 (m, 7H), 7.63 (d, 4H), 7.81 (d, 4H), 7.98 (s, 1H), 8.14 (m, 3H)

### <Examples 4 to 6 and Comparative Examples 1 and 2>

### (Fabrication and Evaluation of Organic Electroluminescence Devices)

A film was formed by spin coating by use of a solution of poly(ethylenedioxythiophene)/poly(styrenesulfonic acid) (manufactured by H. C. Starck Ltd., trade name: Baytron AI4083) on a glass substrate which had an indium-tin oxide (ITO) film with a thickness of 150 nm formed thereon by a sputtering method. The film was dried in air on a hot plate at 200°C for 10 minutes. Thus each hole injection layer (film thickness: 60 nm) was formed. Next, a toluene solution of Polymer Compound 2 (filtered through a 0.2-µm Teflon (registered trademark) filter) was applied by spin coating. The substrate was baked in a glove box under nitrogen atmosphere at 200°C for 15 minutes. Thus each hole transporting layer (film thickness: 20 nm) was formed. Further, each toluene solution (filtered through a 0.2-µmTeflon (registered trademark) filter) was prepared according to the corresponding conditions (the presence or absence of the compound, the kind of the compound, and the composition) shown in Table 1. The obtained solution was applied by spin coating to form a light emitting layer. Adjustment was made, so that the film thickness of the light emitting layer was 70 nm.

This was dried under vacuum at 90°C for 1 hour. Thereafter, LiF was vapor-deposited in 4 nm, and then Al was vapor-deposited in 100 nm. During these vapor depositions, the degree of vacuum was in the range of 1×10⁻⁴ Pa to 9×10⁻³ Pa. The shape of each device was a 2 mm × 2 mm square. A voltage which was changed stepwise was applied to each of the obtained devices to determine the current density and the luminance of the light emission. Table 1 shows the current densities at a bias voltage of 6 V and the driving voltages at a luminance of 1000 cd/m²●hour. Note that EL emission of all of the devices was blue light emission having the peak of the light-emission intensity at 470 nm.

**[Table 1]**

| | Light emitting layer | Current density (mA/cm²) | Driving voltages (V) at 1000 cd/m²●hour |
|---|---|---|---|
| Example 4 | Polymer Compound 1/Compound H = 100/40 (weight ratio) | 84 | 3.9 |
| Example 5 | Polymer Compound 1/Compound J = 100/40 (weight ratio) | 58 | 4.2 |
| Example 6 | Polymer Compound 1/Compound L = 100/40 (weight ratio) | 210 | 3.5 |
| Comparative Example 1 | Polymer Compound 1 | 41 | 4.9 |
| Comparative Example 2 | Polymer Compound 1/CBP = 100/40 (weight ratio) | 46 | 4.6 |

### -Evaluation-

As seen from Table 1, the light emitting layers each formed by using a composition containing the conjugated polymer (Polymer Compound 1) and one of the compounds (Compound H, Compound J, and Compound L) each represented by the Formula (1) improved the current densities at a bias voltage of 6 V of the obtained organic electroluminescence devices, and also lowered the driving voltages at a luminance of 1000 cd/m²●hour, when compared with the light emitting layer formed by using the composition not containing the compound represented by Formula (1) and the light emitting layer containing CBP. Accordingly, it was found that the composition of the present invention has an excellent charge transporting property and charge injection property, and allows low-voltage driving. In addition, the compound of the present invention is useful as a component of the composition. Further, no aggregations of the compound were observed in the solutions of compounds of the present invention, and smooth coating films were obtained with the solutions of compounds of the present invention (in other words, a favorable coating property was observed).

## Claims

1. A compound represented by the following Formula (1): 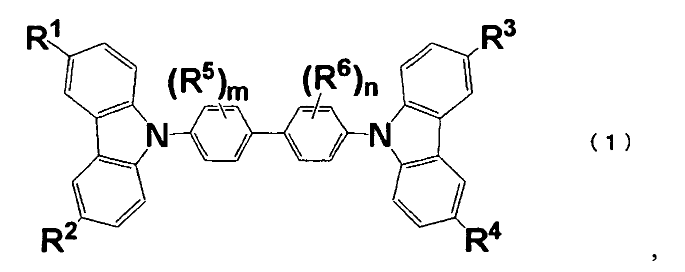 wherein R¹ to R⁴ each independently represent a group selected from the group consisting of a hydrogen atom, an alkyl group having 1 to 18 carbon atoms and a group represented by the following Formula (2): 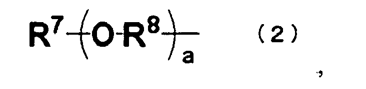 wherein R⁷ represents an alkyl group having 1 to 18 carbon atoms, R⁸ represents an alkylene group having 1 to 20 carbon atoms, a represents an integer of 1 to 5, and R⁸s are either the same as or different from each other when a is 2 or more) ; at least one of R¹ to R⁴ is a group represented by Formula (2); R⁵ and R⁶ each independently represent an alkyl group having 1 to 18 carbon atoms; and m and n each independently represent an integer of 0 to 4).

2. The compound according to claim 1, wherein a is an integer of 2 to 4.

3. The compound according to claim 2, wherein a is 3.

4. A charge transporting material, the material comprising the compound according to any one of claims 1 to 3.

5. A composition comprising:
at least one polymer material selected from a charge transporting material and a light emitting material; and
the compound according to any one of claims 1 to 3.

6. The composition according to claim 5, wherein the polymer material is a conjugated polymer.

7. The composition according to claim 5 or 6, further comprising a solvent, wherein
the composition is in a solution state.

8. The composition according to any one of claims 5 to 7, wherein
a content of the compound in the composition is 0.1 to 10000 parts by weight per 100 parts by weight of the polymer material.

9. An organic transistor comprising:
a charge transporting layer or an active layer made of the composition according to any one of claims 5 to 8.

10. An organic electroluminescence device comprising:
a charge transporting layer or a light emitting layer made of the composition according to any one of claims 5 to 8.

11. An organic solar cell comprising:
a charge transporting layer or an active layer made of the composition according to any one of claims 5 to 8.

## Patentansprüche

1. Eine Verbindung der folgenden Formel (1): 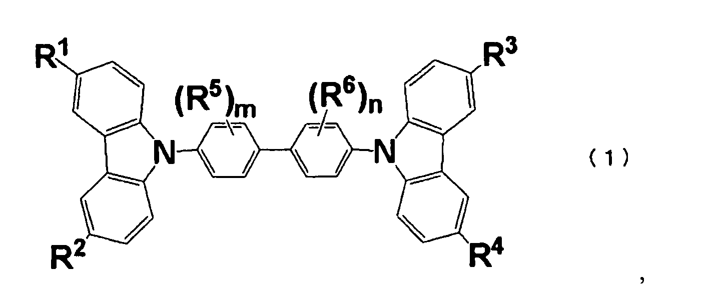 wobei R¹ bis R⁴ jeweils unabhängig einen aus der Gruppe bestehend aus einem Wasserstoffatom, einem Alkylrest mit 1 bis 18 Kohlenstoffatomen und einem Rest der folgenden Formel (2) ausgewählten Rest darstellen: 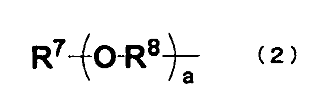 wobei R⁷ einen Alkylrest mit 1 bis 18 Kohlenstoffatom darstellt, R⁸ einen Alkylenrest mit 1 bis 20 Kohlenstoffatomen darstellt, a eine ganze Zahl von 1 bis 5 darstellt und Reste R⁸ entweder gleich oder voneinander verschieden sind, wenn a gleich 2 oder mehr ist; mindestens ein Rest R¹ bis R⁴ ein Rest der Formel (2) ist; R⁵ und R⁶ jeweils unabhängig einen Alkylrest mit 1 bis 18 Kohlenstoffatomen darstellen; und m und n jeweils unabhängig eine ganze Zahl von 0 bis 4 darstellen.

2. Die Verbindung gemäß Anspruch 1, wobei a eine ganze Zahl von 2 bis 4 ist.

3. Die Verbindung gemäß Anspruch 2, wobei a gleich 3 ist.

4. Ein Ladung transportierendes Material, wobei das Material die Verbindung gemäß einem der Ansprüche 1 bis 3 umfasst.

5. Eine Zusammensetzung, umfassend:
mindestens ein aus einem Ladung transportierenden Material und einem Licht emittierenden Material ausgewähltes Polymermaterial; und die Verbindung gemäß einem der Ansprüche 1 bis 3.

6. Die Zusammensetzung gemäß Anspruch 5, wobei das Polymermaterial ein konjugiertes Polymer ist.

7. Die Zusammensetzung gemäß Anspruch 5 oder 6, weiter umfassend ein Lösungsmittel, wobei sich die Zusammensetzung in einem Lösungszustand befindet.

8. Die Zusammensetzung gemäß einem der Ansprüche 5 bis 7, wobei ein Gehalt der Verbindung in der Zusammensetzung 0,1 bis 10000 Gewichtsteile, bezogen auf 100 Gewichtsteile des Polymermaterials, beträgt.

9. Ein organischer Transistor, umfassend:
eine Ladung transportierende Schicht oder eine aktive Schicht, hergestellt aus der Zusammensetzung gemäß einem der Ansprüche 5 bis 8.

10. Eine organische Elektrolumineszenzanordnung, umfassend:
eine Ladung transportierende Schicht oder eine Licht emittierende Schicht, hergestellt aus der Zusammensetzung gemäß einem der Ansprüche 5 bis 8.

11. Eine organische Solarzelle, umfassend:
eine Ladung transportierende Schicht oder eine aktive Schicht, hergestellt aus der Zusammensetzung gemäß einem der Ansprüche 5 bis 8.

## Revendications

1. Composé représenté par la formule (1) suivante : 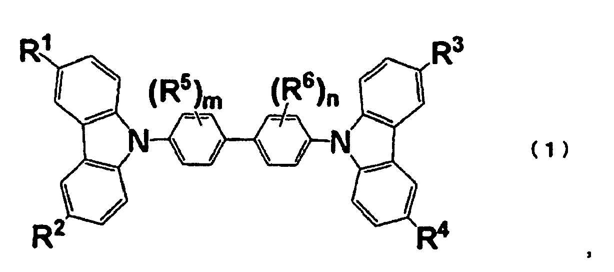 dans laquelle R¹ à R⁴ représentent chacun indépendamment un groupe choisi dans le groupe constitué d'un atome d'hydrogène, d'un groupe alkyle ayant de 1 à 18 atomes de carbone et d'un groupe représenté par la formule (2) suivante : 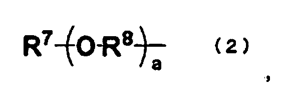 dans laquelle R⁷ représente un groupe alkyle ayant de 1 à 18 atomes de carbone, R⁸ représente un groupe alkylène ayant de 1 à 20 atomes de carbone, a représente un nombre entier de 1 à 5, et les R⁸ sont soit identiques, soit différents l'un de l'autre lorsque a est égal à 2 ou supérieur ; au moins un de R¹ à R⁴ est un groupe représenté par la formule (2) ; R⁵ et R⁶ représentent chacun indépendamment un groupe alkyle ayant de 1 à 18 atomes de carbone ; et m et n représentent chacun indépendamment un nombre entier de 0 à 4.

2. Composé selon la revendication 1, dans lequel a est un nombre entier de 2 à 4.

3. Composé selon la revendication 2, dans lequel a est égal à 3.

4. Matériau de transport de charge, le matériau comprenant le composé selon l'une quelconque des revendications 1 à 3.

5. Composition comprenant :
au moins un matériau polymère choisi parmi un matériau de transport de charge et un matériau émettant de la lumière ; et
le composé selon l'une quelconque des revendications 1 à 3.

6. Composition selon la revendication 5, dans laquelle le matériau polymère est un polymère conjugué.

7. Composition selon la revendication 5 ou 6 comprenant de plus un solvant, dans laquelle
la composition est dans un état de solution.

8. Composition selon l'une quelconque des revendications 5 à 7, dans laquelle
une teneur du composé dans la composition est de 0,1 à 10 000 parties en masse pour 100 parties en masse du matériau polymère.

9. Transistor organique comprenant :
une couche de transport de charge ou une couche active constituée de la composition selon l'une quelconque des revendications 5 à 8.

10. Dispositif d'électroluminescence organique comprenant :
une couche de transport de charge ou une couche émettant de la lumière constituée de la composition selon l'une quelconque des revendications 5 à 8.

11. Cellule photovoltaïque organique comprenant :
une couche de transport de charge ou une couche active constituée de la composition selon l'une quelconque des revendications 5 à 8.
